# EUROPÄISCHE PATENTSCHRIFT

(11) **EP 2 539 382 B1**
(45) Veröffentlichungstag und Bekanntmachung des Hinweises auf die Patenterteilung: **22.10.2014**
(21) Anmeldenummer: 11703696.2
(22) Anmeldetag: 16.02.2011
(51) Int. Cl.: C08F 220/06, A61L 15/12, C08F 6/00

(54) **VERFAHREN ZUR HERSTELLUNG WASSERABSORBIERENDER POLYMERPARTIKEL**
METHOD FOR PRODUCING WATER-ABSORBING POLYMER PARTICLES
PROCÉDÉ POUR PRODUIRE DES PARTICULES DE POLYMÈRES HYDROABSORBANTES

(30) Priorität: 24.02.2010 EP 10154513
(43) Veröffentlichungstag der Anmeldung: 02.01.2013
(73) Patentinhaber: BASF SE, 67056 Ludwigshafen (DE)
(72) Erfinder: FUNK, Rüdiger, 65527 Niedernhausen (DE); SCHRÖDER, Jürgen, 67071 Ludwigshafen (DE); PFEIFFER, Thomas, 67459 Böhl-Iggelheim (DE); WEISMANTEL, Matthias, 63637 Jossgrund (DE); BLEI, Stefan, 68199 Mannheim (DE)
(86) Internationale Anmeldenummer: PCT/EP2011/052284
(87) Internationale Veröffentlichungsnummer: WO 2011/104152

(56) Entgegenhaltungen:
- WO-A1-2006/100300
- WO-A1-2008/087114

## Beschreibung

Die vorliegende Erfindung betrifft ein Verfahren zur Herstellung wasserabsorbierender Polymerpartikel, wobei ein wässriges Polymergel in einem Umluftbandtrockner auf einem umlaufenden Förderband getrocknet wird und die Oberfläche des Förderbandes eine Rauheit von mindestens 0,9 µm aufweist.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die durch Polymerisation erhaltenen wässrigen Polymergele werden typischerweise mittels eines Umluftbandtrockners getrocknet. Problematisch ist hierbei die Schrumpfung des Polymergels auf dem Förderband des Umluftbandtrockners. Dies führt dazu, dass das Förderband mit fortschreitender Trocknung nicht mehr über die gesamte Förderbandbreite mit Polymergel belegt ist und an den Randbereichen ein Teil der Trocknungsluft ungenutzt durch das Förderband strömt (by-pass).

Zur Lösung dieses Problems wird die Verwendung mehrstufiger Umluftbandtrockner vorgeschlagen, wobei das Trocknungsgut auf dem jeweils nächsten Förderband neu verteilt wird (siehe auch in "Perry's Chemical Engineers' Handbook", 7. Auflage, McGraw-Hill, Seite 12-48).

Aufgabe der vorliegenden Erfindung war die Bereitstellung eines verbesserten Verfahrens zur Trocknung wässriger Polymergele mittels eines Umluftbandtrockners.

Gelöst wurde die Aufgabe durch ein Verfahren zur Herstellung wasserabsorbierender Polymerpartikel durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

umfassend Trocknung des erhaltenen wässrigen Polymergels in einem Umluftbandtrockner mittels eines umlaufenden Förderbandes, Mahlung, Klassierung, und optional thermische Oberflächennachvernetzung, dadurch gekennzeichnet, dass die Oberfläche des umlaufenden Förderbandes eine Rauheit R_{z} von mindestens 0,9 µm aufweist.

Die Oberfläche des umlaufenden Förderbandes weist eine Rauheit R_{z} von vorzugsweise mindestens 0,95 µm, besonders bevorzugt von mindestens 1 µm, ganz besonders bevorzugt von mindestens 2 µm, auf. Die Rauheit R_{z} ist die größte Höhe des Profils und wird in der DIN EN ISO 4287 beschrieben.

Der vorliegenden Erfindung liegt die Erkenntnis zugrunde, dass die Schrumpfung der Polymergele während der Trocknung durch die Auswahl geeigneter Oberflächen vermindert werden kann, ohne dass es zu einer vermehrten Rissbildung kommt. Die Ursache dafür ist vermutlich, dass das zu trocknende Polymergel während der Trocknung eine Temperatur oberhalb der Glasübergangstemperatur T_{G} aufweist. Oberhalb der Glasübergangstemperatur T_{G} sind Polymere gummielastisch und klebrig. Somit sind die Polymerpartikel aufgrund ihrer Klebrigkeit in der Lage an der Oberfläche des Förderbandes zu kleben, die auftretenden Zugkräfte aufzunehmen und die Rissbildung zu verhindern. Dieses Kleben auf der Oberfläche wird durch eine große Rauheit R_{z} begünstigt.

Für das erfindungsgemäße Verfahren geeignete Umluftbandtrockner werden beispielsweise in der Monographie "Modern Superabsorbent Polymer Technology", F.L. Buchholz und A.T. Graham, Wiley-VCH, 1998, Seiten 89 bis 92, beschrieben.

Vorteilhaft werden im erfindungsgemäßen Verfahren Umluftbandtrockner mit Förderbändern eingesetzt, deren produktberührte Oberflächen aus einem nichtrostenden Stahl sind. Hierbei kann die Rauheit R_{z} durch eine entsprechende Oberflächenbehandlung auf den gewünschten Wert eingestellt werden, beispielsweise durch sandstrahlen. Sandgestrahlte Stahloberflächen weisen eine größere Rauheit R_{z} auf als matte oder polierte Stahloberflächen.

Nichtrostende Stähle weisen üblicherweise einen Chromgehalt von 10,5 bis 13 Gew.-% Chrom auf. Der hohe Chromanteil führt zu einer schützenden Passivierung aus Chromdioxid an der Stahloberfläche. Weitere Legierungsbestandteile erhöhen die Korrosionsbeständigkeit und verbessern die mechanischen Eigenschaften.

Besonders geeignete Stähle sind austenitische Stähle mit beispielsweise mindestens 0,08 Gew.-% Kohlenstoff. Vorteilhaft enthalten die austenitischen Stähle neben Eisen, Kohlenstoff, Chrom, Nickel und optional Molybdän noch weitere Legierungsbestandteile, vorzugsweise Niob oder Titan.

Die bevorzugten nichtrostenden Stähle sind Stähle mit der Werkstoffnummer 1.43xx oder 1.45xx gemäß der DIN EN 10020, wobei xx eine natürliche Zahl zwischen 0 und 99 sein kann. Besonders bevorzugte Werkstoffe sind die Stähle mit den Werkstoffnummern 1.4301, 1.4541 und 1.4571, insbesondere Stahl mit der Werkstoffnummer 1.4301.

Das umlaufende Förderband weist üblicherweise eine Vielzahl von Öffnungen auf. In einer bevorzugten Ausführungsform der vorliegenden Erfindung weist das umlaufende Förderband quer zur Transportrichtung eine Vielzahl von in versetzten Reihen angeordneten Schlitzen mit einer Länge von vorzugsweise 5 bis 50 mm, besonders bevorzugt 10 bis 40 mm, ganz besonders bevorzugt 15 bis 30 mm, einer Breite von vorzugsweise 0,5 bis 5 mm, besonders bevorzugt 1 bis 4 mm, ganz besonders bevorzugt 1,5 bis 3 mm, und einem Verhältnis von Länge zu Breite von vorzugsweise 2 bis 20, besonders bevorzugt 5 bis 15, ganz besonders bevorzugt 8 bis 12, aufweist.

Die Breite des Umluftbandtrockners beträgt vorzugsweise von 1 bis 10 m, besonders bevorzugt von 2 bis 7,5 m, ganz besonders bevorzugt von 3 bis 5 m.

Die Länge des Umluftbandtrockners beträgt vorzugsweise von 10 bis 80 m, besonders bevorzugt von 30 bis 60 m, ganz besonders bevorzugt von 40 bis 50 m.

Die Förderbandgeschwindigkeit des Umluftbandtrockners beträgt vorzugsweise von 0,005 bis 0,05 m/s, besonders bevorzugt von 0,01 bis 0,35 m/s, ganz besonders bevorzugt von 0,015 bis 0,025 m/s.

Die Verweilzeit auf dem Umluftbandtrockner beträgt vorzugsweise von 10 bis 120 Minuten, besonders bevorzugt von 20 bis 90 Minuten, ganz besonders bevorzugt von 30 bis 60 Minuten.

Der Wassergehalt der Polymergelschüttung in der Aufgabezone beträgt vorzugsweise von 25 bis 90 Gew.-%, besonders bevorzugt von 35 bis 70 Gew.-%, ganz besonders bevorzugt von 40 bis 60 Gew.-%. Die mittlere Partikelgröße des wässrigen Polymergels beträgt vorzugsweise von 0,1 bis 10 mm, besonders bevorzugt von 0,5 bis 5 mm, ganz besonders bevorzugt von 1 bis 2 mm.

Die Höhe der Polymergelschüttung auf dem Förderband des Umluftbandtrockners beträgt in der Aufgabezone vorzugsweise von 2 bis 20 cm, besonders bevorzug von 5 bis 15 cm, ganz besonders bevorzugt von 8 bis 12 cm.

Die Gaseingangstemperaturen des Umluftbandtrockners betragen vorzugsweise von 150 bis 200°C, besonders bevorzugt von 160 bis 190°C, ganz besonders bevorzugt von 170 bis 180°C.

Der zur Trocknung verwendete Gasstrom kann Wasserdampf enthalten. Der Wasserdampfanteil sollte aber einen Wert, der einem Taupunkt von vorzugsweise höchstens 50°C, besonders bevorzugt höchstens 40°C, ganz besonders bevorzugt höchstens 30°C, entspricht, nicht übersteigen.

Der Wassergehalt des Polymergels nach der Trocknung auf dem Umluftbandtrockner beträgt vorzugsweise von 0,5 bis 15 Gew.-%, besonders bevorzugt von 1 bis 10 Gew.-%, ganz besonders bevorzugt von 2 bis 8 Gew.-%.

Ein besonders vorteilhaftes Trocknungsverfahren wird in WO 2001/100300 A1 beschrieben.

Im Folgenden wird die Herstellung der wasserabsorbierenden Polymerpartikel näher erläutert:
Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm² (AUL0.3psi) durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende wässrige Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein wässriges Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte wässrige Polymergel zusätzlich extrudiert werden.

Die Säuregruppen der erhaltenen wässrigen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden wässrigen Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des wässrigen Polymergels eingestellt wird. Wird das wässrige Polymergel zumindest teilweise nach der Polymerisation neutralisiert, so wird das wässrige Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das wässrige Polymergel wird dann mit einem Umluftbandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des wässrigen Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene wässrige Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl während als auch nach der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizöntalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die O-berflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die Temperatur der wasserabsorbierenden Polymerpartikel im Trockner beträgt vorzugsweise von 100 bis 250°C, besonders bevorzugt von 130 bis 220°C, ganz besonders bevorzugt von 150 bis 200°C. Die Verweilzeit im Trockner beträgt vorzugsweise von 10 bis 120 Minuten, besonders bevorzugt von 10 bis 90 Minuten, ganz besonders bevorzugt von 30 bis 60 Minuten. Der Füllgrad des Trockners beträgt vorzugsweise von 30 bis 80%, besonders bevorzugt von 40 bis 75%, ganz besonders bevorzugt von 50 bis 70%. Der Füllgrad des Trockners kann über die Höhe des Ablaufwehrs eingestellt werden.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Feuchtegehalt gemäß der von der EDANA empfohlenen Testmethode Nr. WSP 230.2-05 "Moisture Content" bestimmt wird.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Centrifuge Retention Capacity" bestimmt.

### Beispiele

### Beispiel 1 (Herstellung des wässrigen Polymergels)

4.485 g einer 37,3gew.-%igen wässrigen Natriumacrylatlösung wurden mit 427 g Acrylsäure und 1.024 g Wasser gemischt und mit Stickstoff inertisiert. Diese Mischung wurde in einen mit Stickstoff inertisierten Werner & Pfleiderer LUK 8,0 K2 Kneter (2 Sigma-Wellen) eingefüllt und nacheinander mit 11,96 g 15-fach ethoxyliertem Trimethylolpropantriacrylat, 20,50 g einer 0,5gew.-%igen wässrigen Ascorbinsäurelösung und 31,66 g einer 15gew.-%igen wässrigen Natriumpersulfatlösung versetzt. Der Kneter wurde bei Maximaldrehzahl (ca. 98 upm der schnelleren Welle, ca. 49 upm der langsameren Welle, Verhältnis ca. 2:1) gerührt. Sofort nach der Zugabe von Natriumpersulfat wurde der Knetermantel mit 74°C warmen Wärmeträger beheizt. Nach Erreichen der Maximaltemperatur wurde die Mantelheizung abgeschaltet und im Kneter weitere 15 Minuten nachreagieren lassen. Das erhaltene Polymergel wurde auf 63°C abgekühlt, ausgeleert und für die folgenden Versuche verwendet.

### Beispiel 2 (Vergleichsbeispiel)

1.234 g wässriges Polymergel aus Beispiel 1 wurden in eine Trocknungswanne eingefüllt und 370 Minuten bei 175°C in einem Umlufttrockenschrank getrocknet. Die Trocknungswanne bestand aus einem 5 cm hohen Edelstahlrahmen mit einem Innenmaß von 23,7 x 23,7 cm und einer auswechselbaren Edelstahlbodenplatte (Werkstoffnummer 1.4301). Die Edelstahlbodenplatte war quadratisch, hatte parallele Reihen von Schlitzen mit einer Öffnung von 2 x 20 mm und wurde passgenau in den Edelstahlrahmen eingelegt.

Die Oberfläche der Edelstahlbodenplatte hatte eine Rauheit R_{z} von 0,32 µm.

Der Edelstahlrahmen wurde vorher mit PTFE-Spray eingesprüht, um mögliche Anhaftungen zu vermeiden. Das wässrige Polymergel in der Trocknungswanne wurde vor der Trocknung mit einer passenden Edelstahlplatte beschwert, um zu verhindern, dass sich die Ränder des Polymergels beim Trocknen hochbiegen. Die Edelstahlplatte hatte parallele Reihen von Schlitzen mit einer Öffnung von 2 x 20 mm.

Das Polymergel schrumpfte bei der Trocknung dreidimensional. Für die Auswertung der Schrumpfung wurde nur die zweidimensionale bzw. flächige Schrumpfung ermittelt. Dazu wurde das getrocknete Polymergel als kompletter Block auf eine schwarze Unterlage gelegt, aus einer Entfernung von 56 cm von oben fotografiert und die prozentuale Schrumpfung über eine Pixelauswertung erfasst.

Um Alterungsprozeße des Polymergels und andere Störgrößen auszuschließen wurde parallel eine Trocknung mit einer Edelstahlbodenplatte mit einer Rauheit R_{z} von 1,03 µm durchgeführt und die Schrumpfung dieser Kontrollmessung zu 100% gesetzt.

Die Schrumpfung der untersuchten Edelstahlbodenplatte mit einer Rauheit R_{z} von 0,32 µm betrug 95%. Das getrocknete Polymergel ließ sich nur sehr schwer von der Edelstahlbodenplatte lösen.

### Beispiel 3 (Vergleichsbeispiel)

Es wurde verfahren wie unter Beispiel 2. Die Edelstahlbodenplatte wurde ausgetauscht.

Die Oberfläche der Edelstahlbodenplatte hatte eine Rauheit R_{z} von 0,87 µm.

Die Schrumpfung der untersuchten Edelstahlbodenplatte mit einer Rauheit R_{z} von 0,87 µm betrug 152%. Das getrocknete Polymergel ließ sich leicht von der Edelstahlbodenplatte lösen.

### Beispiel 4

Es wurde verfahren wie unter Beispiel 2. Die Edelstahlbodenplatte wurde ausgetauscht.

Die Oberfläche der Edelstahlbodenplatte hatte eine Rauheit R_{z} von 7,32 µm.

Die Schrumpfung der untersuchten Edelstahlbodenplatte mit einer Rauheit R_{z} von 7,32 µm betrug 92%. Das getrocknete Polymergel ließ sich leicht von der Edelstahlbodenplatte lösen.

### Beispiel 5

Es wurde verfahren wie unter Beispiel 2. Die Edelstahlbodenplatte wurde ausgetauscht.

Die Oberfläche der Edelstahlbodenplatte hatte eine Rauheit R_{z} von 18,4 µm.

Die Schrumpfung der untersuchten Edelstahlbodenplatte mit einer Rauheit R_{z} von 18,4 µm betrug 67%. Das getrocknete Polymergel ließ sich leicht von der Edelstahlbodenplatte lösen.

## Patentansprüche

1. Verfahren zur Herstellung wasserabsorbierender Polymerpartikel durch Polymerisation einer wässrigen Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
umfassend Trocknung des erhaltenen wässrigen Polymergels in einem Umluftbandtrockner mittels eines umlaufenden Förderbandes, Mahlung, Klassierung, und optional thermische Oberflächennachvernetzung, **dadurch gekennzeichnet, dass** die Oberfläche des umlaufenden Förderbandes eine Rauheit R_{z} von mindestens 0,9 µm aufweist.

2. Verfahren gemäß Anspruch 1, **dadurch gekennzeichnet, dass** das umlaufende Förderband aus austenitischem Stahl ist.

3. Verfahren gemäß Anspruch 1 oder 2, **dadurch gekennzeichnet, dass** das umlaufende Förderband eine Vielzahl von Öffnungen aufweist.

4. Verfahren gemäß einem der Ansprüche 1 bis 3, **dadurch gekennzeichnet, dass** das umlaufende Förderband quer zur Transportrichtung eine Vielzahl von in versetzten Reihen angeordneten Schlitzen mit einer Länge von 5 bis 50 mm, einer Breite von 0,5 bis 5 mm und einem Verhältnis von Länge zu Breite von 2 bis 20 aufweist.

5. Verfahren gemäß einem der Ansprüche 1 bis 4, **dadurch gekennzeichnet, dass** das umlaufende Förderband eine Breite von mindestens 1 m aufweist.

6. Verfahren gemäß einem der Ansprüche 1 bis 5, **dadurch gekennzeichnet, dass** die Förderbandgeschwindigkeit von 0,005 bis 0,05 m/s beträgt.

7. Verfahren gemäß einem der Ansprüche 1 bis 6, **dadurch gekennzeichnet, dass** der Wassergehalt des Polymergels vor der Trocknung im Umluftbandtrockner von 30 bis 70 Gew.-% beträgt.

8. Verfahren gemäß einem der Ansprüche 1 bis 7, **dadurch gekennzeichnet, dass** der Wassergehalt des Polymergels nach der Trocknung im Umluftbandtrockner von 0,5 bis 15 Gew.-% beträgt.

9. Verfahren gemäß einem der Ansprüche 1 bis 8, **dadurch gekennzeichnet, dass** die Höhe der Polymergelschüttung auf dem umlaufenden Förderband von 2 bis 20 cm beträgt.

10. Verfahren gemäß einem der Ansprüche 1 bis 9, **dadurch gekennzeichnet, dass** die wasserabsorbierenden Polymerpartikel eine Zentrifugenretentionskapazität von mindestens 15 g/g aufweisen.

## Claims

1. A process for producing water-absorbing polymer particles by polymerizing an aqueous monomer solution or suspension comprising
a) at least one ethylenically unsaturated monomer which bears acid groups and may be at least partly neutralized,
b) at least one crosslinker,
c) at least one initiator,
d) optionally one or more ethylenically unsaturated monomers copolymerizable with the monomers mentioned under a) and
e) optionally one or more water-soluble polymers,
comprising drying of the resulting aqueous polymer gel in a forced-air belt drier by means of a circulating conveyor belt, grinding, classifying and optional thermal surface postcrosslinking, wherein the surface of the circulating conveyor belt has a roughness R_{z} of at least 0.9 µm.

2. The process according to claim 1, wherein the circulating conveyor belt is made from austenitic steel.

3. The process according to claim 1 or 2, wherein the circulating conveyor belt has a multitude of orifices.

4. The process according to any of claims 1 to 3, wherein the circulating conveyor belt has, transverse to the direction of transport, a multitude of slots arranged in offset rows and having a length of 5 to 50 mm, a width of 0.5 to 5 mm and a ratio of length to width of 2 to 20.

5. The process according to any of claims 1 to 4, wherein the circulating conveyor belt has a width of at least 1 m.

6. The process according to any of claims 1 to 5, wherein the conveyor belt speed is from 0.005 to 0.05 m/s.

7. The process according to any of claims 1 to 6, wherein the water content of the polymer gel before the drying in the forced-air belt drier is from 30 to 70% by weight.

8. The process according to any of claims 1 to 7, wherein the water content of the polymer gel after the drying in the forced-air belt drier is from 0.5 to 15% by weight.

9. The process according to any of claims 1 to 8, wherein the height of the polymer gel bed on the circulating conveyor belt is from 2 to 20 cm.

10. The process according to any of claims 1 to 9, wherein the water-absorbing polymer particles have a centrifuge retention capacity of at least 15 g/g.

## Revendications

1. Procédé de fabrication de particules polymères absorbant l'eau par polymérisation d'une solution ou suspension aqueuse de monomères, contenant :
a) au moins un monomère éthyléniquement insaturé, portant des groupes acides, qui peut être au moins partiellement neutralisé,
b) au moins un agent de réticulation,
c) au moins un initiateur,
d) éventuellement un ou plusieurs monomères éthyléniquement insaturés copolymérisables avec les monomères mentionnés en a), et
e) éventuellement un ou plusieurs polymères solubles dans l'eau,
comprenant un séchage du gel polymère aqueux obtenu dans un séchoir à bande à circulation d'air au moyen d'une bande transporteuse en circulation, un broyage, une classification et éventuellement une post-réticulation de surface thermique, **caractérisé en ce que** la surface de la bande transporteuse en circulation présente une rugosité R_{z} d'au moins 0,9 µm.

2. Procédé selon la revendication 1, **caractérisé en ce que** la bande transporteuse en circulation est en acier austénitique.

3. Procédé selon la revendication 1 ou 2, **caractérisé en ce que** la bande transporteuse en circulation comprend une pluralité d'ouvertures.

4. Procédé selon l'une quelconque des revendications 1 à 3, **caractérisé en ce que** la bande transporteuse en circulation comprend perpendiculairement à la direction de transport une pluralité de fentes agencées en séries décalées, d'une longueur de 5 à 50 mm, d'une largeur de 0,5 à 5 mm et d'un rapport longueur sur largeur de 2 à 20.

5. Procédé selon l'une quelconque des revendications 1 à 4, **caractérisé en ce que** la bande transporteuse en circulation présente une largeur d'au moins 1 m.

6. Procédé selon l'une quelconque des revendications 1 à 5, **caractérisé en ce que** la vitesse de la bande transporteuse est de 0,005 à 0,05 m/s.

7. Procédé selon l'une quelconque des revendications 1 à 6, **caractérisé en ce que** la teneur en eau du gel polymère avant le séchage dans le séchoir à bande à circulation d'air est de 30 à 70 % en poids.

8. Procédé selon l'une quelconque des revendications 1 à 7, **caractérisé en ce que** la teneur en eau du gel polymère après le séchage dans le séchoir à bande à circulation d'air est de 0,5 à 15 % en poids.

9. Procédé selon l'une quelconque des revendications 1 à 8, **caractérisé en ce que** la hauteur du garnissage de gel polymère sur la bande transporteuse en circulation est de 2 à 20 cm.

10. Procédé selon l'une quelconque des revendications 1 à 9, **caractérisé en ce que** les particules polymères absorbant l'eau présentent une capacité de rétention centrifuge d'au moins 15 g/g.
